# DEMANDE DE BREVET EUROPEEN

(11) **EP 1 312 341 A2**
(43) Date de publication de la demande: **21.05.2003**
(21) Numéro de dépôt: 02292662.0
(22) Date de dépôt: 25.10.2002
(51) Int. Cl.: A61K 7/13, C08L 83/08

(54) **Utilisation de silicones aminés particulières en pré-traitement de colorations directe ou d'oxydation de fibres kératiniques**

(30) Priorité: 08.11.2001 FR 0114484
(71) Demandeur: L'OREAL, 75008 Paris (FR)
(72) Inventeur: Devin-Baudoin, Priscille, 92170 Vanves (FR); Sabbagh, Anne, 92500 Rueil Malmaison (FR)
(74) Mandataire: Wattremez, Catherine

(57) **Abrégé**

La présente invention a pour objet l'utilisation, en pré-traitement d'une coloration directe ou d'oxydation des fibres kératiniques humaines et plus particulièrement des cheveux, d'une composition comprenant au moins une silicone aminée particulière.

Elle a aussi pour objet un procédé de coloration directe ou d'oxydation des fibres kératiniques humaines et plus particulièrement des cheveux comprenant un pré-traitement avec une composition comprenant au moins une silicone aminée particulière.

## Description

La présente invention a pour objet l'utilisation, en pré-traitement d'une coloration directe ou d'oxydation des fibres kératiniques humaines et plus particulièrement des cheveux, d'une composition comprenant au moins une silicone aminée particulière.
Elle a aussi pour objet un procédé de coloration directe ou d'oxydation des fibres kératiniques humaines et plus particulièrement des cheveux comprenant un pré-traitement avec une composition comprenant au moins une silicone aminée particulière.

Il existe principalement deux types de coloration des fibres kératiniques. La coloration directe, mettant en oeuvre en présence ou en l'absence d'agents oxydants, des colorants directs et / ou des pigments qui sont des molécules colorées, conférant aux fibres une couleur temporaire qui s'estompe après quelques shampooings, et la coloration dite "coloration d'oxydation" mettant en oeuvre des précurseurs de colorants d'oxydation et un agent oxydant, qui confère aux fibres une couleur plus tenace que la précédente.

Il existe un besoin d'améliorer la montée de ces colorations sur les fibres, en particulier sur des fibres sensibilisées car celles-ci sont plus poreuses et fixent moins les colorants.
Par ailleurs, l'utilisation d'un agent oxydant entraîne en général une certaine dégradation de la fibre kératinique.
Il existe donc un besoin de limiter ces dégradations et les conséquences qu'elles entraînent sur l'état cosmétique de la fibre.

Après d'importantes études sur la question, la demanderesse a découvert de manière tout à fait inattendue et surprenante que l'utilisation, en pré-traitement sur des fibres kératiniques humaines et plus particulièrement des cheveux, d'une composition comprenant au moins une silicone aminée particulière, permettait de résoudre ces problèmes.
Cette découverte est à l'origine de la présente invention.

En outre, ce pré-traitement améliore la ténacité des colorations directes ou d'oxydation, en particulier vis à vis des shampooings.

Un premier objet de l'invention concerne donc l'utilisation, en pré-traitement d'une coloration d'oxydation ou d'une coloration directe des fibres kératiniques humaines et plus particulièrement des cheveux, d'une composition comprenant au moins une silicone aminée aminoéthyliminoalkyl(C₄-C₈).

Ladite utilisation a notamment pour objet d'améliorer la montée de la couleur, en particulier sur des cheveux sensibilisés et/ou l'état de la fibre après coloration, notamment dans le cas de coloration avec oxydant ainsi que la ténacité aux shampooings desdites colorations.

Par amélioration de l'état de la fibre on entend une diminution de la porosité ou de la solubilité alcaline de la fibre et une amélioration des propriétés cosmétiques et en particulier du lissage, de la douceur et de la facilité de démêlage et de coiffage.
Cet effet est rémanent, c'est à dire durable.
La porosité se mesure par la fixation à 37°C et à pH10, en 2 minutes, de la 2-nitro paraphénylènediamine à 0,25% dans un mélange éthanol / tampon pH 10 (rapport volumique 10/90).
La solubilité alcaline correspond à la perte de masse d'un échantillon de 100 mg de fibres kératiniques sous l'action de la soude décinormale pendant 30 minutes à 65°C.

Un second objet de l'invention concerne un procédé de coloration consistant à appliquer sur les fibres kératiniques humaines et plus particulièrement des cheveux, dans une première étape, une composition comprenant au moins une silicone aminée aminoéthyliminoalkyl(C₄-C₈), à rincer ou non rincer les fibres, puis dans une seconde étape, à appliquer une composition colorante directe ou d'oxydation pendant un temps suffisant pour développer la couleur, à faire suivre ou non par un rinçage, puis éventuellement par un shampooing, et ensuite un séchage.

### Silicones aminées

Les silicones aminées selon l'invention présentent la formule suivante : 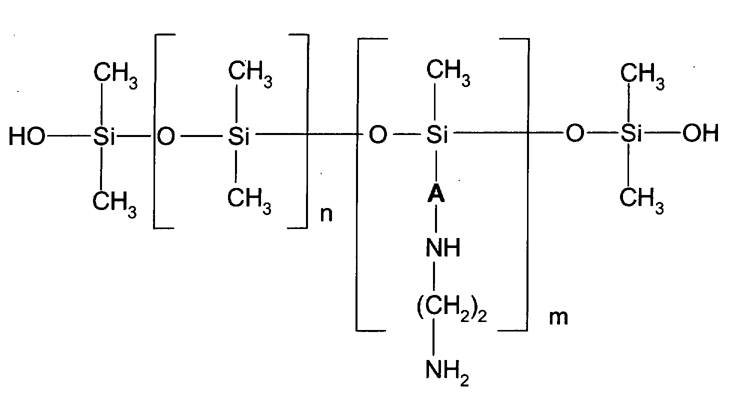 dans laquelle :
**A** désigne un radical alkylène linéaire ou ramifié en C₄-C₈ et de préférence en C₄
m et n sont des nombres tels que la somme (n + m) peut varier notamment de 1 à 2 000 et en particulier de 50 à 150, n pouvant désigner un nombre allant de 0 à 1 999 et notamment de 49 à 149 et m pouvant désigner un nombre allant de 1 à 2 000, et notamment de 1 à 10.

On entend par radical alkylène des groupements hydrocarbonés saturés divalents.

La viscosité du polymère est de préférence supérieure à 25 000 mm²/s à 25°C.
Plus préférentiellement, cette viscosité peut aller de 30 000 à 200 000 mm²/s à 25°C et encore plus préférentiellement de 30 000 à 150 000 mm²/s à 25°C.
La viscosité des silicones est par exemple mesurée selon la norme « ASTM 445 Appendice C ».

La silicone aminée présente une masse moléculaire moyenne en poids allant de préférence de 2000 à 1000000 et encore plus particulièrement de 3500 à 200000.
Les masses moléculaires moyennes en poids de ces silicones aminées sont mesurées par Chromatographie par Perméation de Gel (GPC) à température ambiante en équivalent polystyrène. Les colonnes utilisées sont des colonnes µ styragel. L'éluant est le THF, le débit est de 1 ml/mn. On injecte 200 µl d'une solution à 0,5% en poids de silicone dans le THF. La détection se fait par réfractométrie et UVmétrie.

Lorsque ces silicones aminées sont mises en oeuvre, une forme de réalisation particulièrement intéressante est leur utilisation sous forme d'émulsion huile-dans-eau.

L'émulsion huile-dans-eau peut comprendre un ou plusieurs tensioactifs. Les tensioactifs peuvent être de toute nature mais de préférence cationique et/ou non ionique.

Les particules de silicone dans l'émulsion ont une taille moyenne allant généralement de 3 nm à 500 nanomètres, de préférence de 5 nm à 300 nanomètres, plus particulièrement de 10 nm à 275 nanomètres et encore plus particulièrement de 150 nm à 275 nanomètres.

Une silicone répondant à cette formule est par exemple la DC2-8299® de la société DOW CORNING.

La silicone aminée est utilisée de préférence dans la composition de pré-traitement en une quantité allant de 0,01 à 20% en poids du poids total de la composition. Plus préférentiellement, cette quantité va de 0,1 à 15% en poids et encore plus particulièrement de 0,5 à 10 % en poids.

La composition de pré-traitement peut contenir tous les ingrédients classiquement utilisés en cosmétique et en particulier dans le domaine capillaire. En particulier elle peut contenir des tensioactifs et/ou des polymères additionnels. Ces tensioactifs et ces polymères peuvent être de nature non-ionique, cationique, anionique ou amphotère.
Parmi les polymères additionnels, les silicones aminées autres que celles de l'invention sont particulièrement préférées.

La composition de post traitement présente un pH allant de 2 à 11 et de préférence de 4 à 9.
Elle peut se présenter sous diverses formes telles que lotions, gels, crèmes, shampooings, sticks, mousses, sprays. Pour certaines de ces formes, elle peut être conditionnée en flacon pompe ou dans un récipient aérosol. Dans le cas de l'aérosol, la composition est associée à un agent propulseur qui peut être par exemple un alcane ou un mélange d'alcane, du diméthyl éther, de l'azote, du protoxyde d'azote, du gaz carbonique ou des halgénoalcanes, ainsi que leurs mélanges.
Une forme particulièrement préférée selon l'invention est la forme shampooing.
Dans ce cas, la composition contient au moins un agent tensioactif qui est de préférence anionique. De préférence alors, elle contient un mélange de tensioactifs dont au moins un agent tensioactif anionique, le ou les autres tensioactifs étant préférentiellement non ioniques ou amphotères.

La composition de pré-traitement peut être utilisée en mode rincé ou en mode non-rincé, c'est à dire que son application est suivie ou non d'un rinçage.
Dans le premier cas, le temps de pose de la composition de pré-traitement est compris entre quelques secondes et 60 minutes et de préférence entre 30 secondes et 15 minutes.

La température d'application de la composition de post traitement peut varier de 10°C à 70°C. De préférence l'application s'effectuera entre 10 et 60°C et plus particulièrement à la température ambiante.

La nature et la concentration des colorants présents dans les compositions colorantes n'est pas critique.

Dans le cas des colorations directes ( en présence ou en l'absence d'agents oxydants) les compositions colorantes comprennent au moins un colorant choisi parmi les colorants directs nitrés benzéniques neutres, acides ou cationiques, les colorants directs azoïques ou méthiniques neutres, acides ou cationiques, les colorants directs quinoniques et en particulier anthraquinoniques neutres, acides ou cationiques, les colorants directs aziniques, les colorants directs triarylméthaniques, les colorants directs indoaminiques, les colorants directs naturels ou leurs mélanges.

Dans le cas des colorations d'oxydation, les compositions colorantes comprennent au moins une base d'oxydation.
Les bases d'oxydation sont choisies parmi celles classiquement utilisées en teinture d'oxydation, et parmi lesquelles on peut notamment citer les ortho- et para-phénylènediamines, les bases doubles, les ortho- et para- aminophénols, les bases hétérocycliques ainsi que leurs sels d'addition avec un acide .
Généralement, les compositions colorantes d'oxydation comprennent un ou plusieurs coupleurs.
Les coupleurs utilisables sont ceux classiquement utilisés dans les compositions de teinture d'oxydation, c'est-à-dire des métaphénylènediamines, des métaaminophénols et des métadiphénols, les dérivés mono- ou poly-hydroxylés du naphtalène, le sésamol et ses dérivés et des composés hétérocycliques tels que par exemple les coupleurs indoliques, les coupleurs indoliniques, les coupleurs pyridiniques et leurs sels d'addition avec un acide.

La nature de l'agent oxydant utilisé dans la coloration directe éclaircissante (coloration directe avec un agent oxydant) ou dans la coloration d'oxydation n'est pas critique.
L'agent oxydant est de préférence choisi dans le groupe formé par le peroxyde d'hydrogène, le peroxyde d'urée, les bromates ou ferricyanures de métaux alcalins, les persels tels que les perborates et les persulfates. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Les exemples suivants sont destinés à illustrer l'invention. Celle-ci n'est cependant pas limitée à ces modes de réalisation.

### EXEMPLES

On a préparé les deux compositions de pré-traitement suivantes :
[exprimées en grammes de Matière Active]

| **Composition A** | |
|---|---|
| Phosphate de diamidon de maïs hydroxypropylé | 3 |
| Hydroxyéthylcellulose | 0,6 |
| Huile de ricin hydrogénée oxyéthylénée (40 OE) | 0,5 |
| Polydiméthylsiloxane selon l'invention : DC2-8299® de la société DOW CORNING | 3,5 |
| Parfum | 0,3 |
| Conservateurs | 0,3 |
| Eau déminéralisée q.s.p | 100 |

| **Composition B** | |
|---|---|
| Lauryléthersulfate de sodium à 2,2 moles d'oxyde d'éthylène | 7 |
| Cocoyl bétaïne | 2,5 |
| Distéarate de glycol | 1,5 |
| Polydiméthylsiloxane selon l'invention : DC2-8299® de la société DOW CORNING..... | 1,8 |
| Hydroxyéthylcellulose quaternisé par le chlorure de 2,3-époxypropyltriméthylammonium vendu sous la marque Ucare Polymer JR-400® par Union Carbide | 0,4 |
| Polymère acrylique en émulsion vendu sous la marque Aqua SF1® par Noveon | 0,8 |
| Conservateurs | q.s. |
| Agents de pH q.s.p | pH 5 |
| Eau déminéralisée q.s.p | 100 |

Les compositions A et B ont été appliquées pendant 15 minutes sur des mèches de cheveux naturels à 90% de blancs et sur des mèches de cheveux sensibilisés par une décoloration.
Après rinçage et séchage, on a effectué sur ces mèches ainsi que sur une mèche témoin sans pré-traitement, une coloration pendant 30 minutes.

Résultats : on a constaté :
- que l'état des fibres ayant subi le pré-traitement et la coloration a été meilleur que celui des fibres n'ayant subi que le procédé de coloration, ce qui se traduit par des cheveux plus doux et plus lisses, plus individualisés, plus faciles à démêler et à coiffer ;
- que la montée de la coloration a été meilleure sur les mèches ayant subi le pré-traitement, en particulier dans le cas des mèches de cheveux sensibilisés.
En outre, la ténacité de la couleur aux shampooings a été satisfaisant.

## Revendications

1. Utilisation, en pré-traitement d'une coloration d'oxydation ou d'une coloration directe des fibres kératiniques humaines et plus particulièrement des cheveux, d'une composition comprenant au moins une silicone aminée aminoéthyliminoalkyl(C₄-C₈).

2. Utilisation selon la revendication 1, **caractérisée par le fait que** la silicone aminée est de formule suivante : 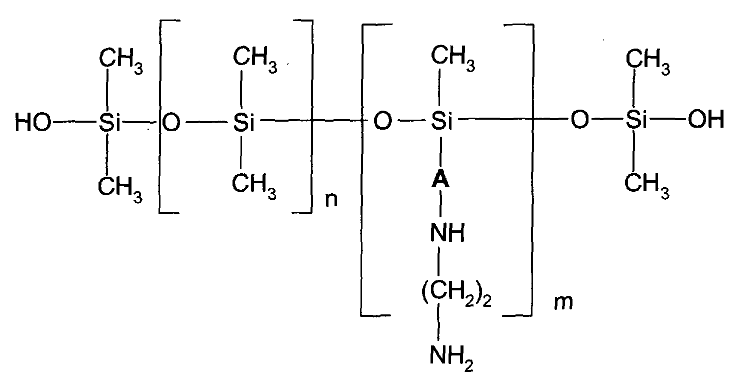 dans laquelle :
**A** désigne un radical alkylène linéaire ou ramifié en C₄-C₈
m et n sont des nombres tels que la somme (n + m) varie de 1 à 2 000 et en particulier de 50 à 150, n désigne un nombre allant de 0 à 1 999 et notamment de 49 à 149 et m désigne un nombre allant de 1 à 2 000, et notamment de 1 à 10.

3. Utilisation selon la revendication 2, **caractérisée par le fait que** A désigne un radical alkylène linéaire ou ramifié en C₄.

4. Utilisation selon l'une quelconque des revendications précédentes, **caractérisée par le fait que** la viscosité du polymère est supérieure à 25 000 mm²/s à 25°C.

5. Utilisation selon la revendication 4, **caractérisée par le fait que** la viscosité va de 30 000 à 200 000 mm²/s à 25°C et plus préférentiellement de 30 000 à 150 000 mm²/s à 25°C.

6. Utilisation selon l'une quelconque des revendications précédentes, **caractérisée par le fait que** la silicone a une masse moléculaire moyenne en poids allant de 2000 à 1000000 et de préférence de 3500 à 200000.

7. Utilisation selon l'une quelconque des revendications précédentes, **caractérisée par le fait que** la silicone aminée est sous forme d'émulsion huile-dans-eau comprenant des agents tensioactifs.

8. Utilisation selon la revendication 7, **caractérisée par le fait que** l'émulsion comprend au moins un agent tensioactif cationique et/ou non ionique.

9. Utilisation selon l'une quelconque des revendications 7 ou 8, **caractérisée par le fait que** les particules de silicone dans l'émulsion ont une taille allant de 3 nm à 500 nanomètres, de préférence de 5 nm à 300 nanomètres, plus particulièrement de 10 nm à 275 nanomètres et encore plus particulièrement de 150 nm à 275 nanomètres.

10. Utilisation selon l'une quelconque des revendications précédentes, **caractérisée par le fait que** la silicone aminée aminoéthyliminoalkyl(C₄-C₈) est présente dans la composition de post traitement en une quantité allant de 0,01 à 20% en poids du poids total de la composition.

11. Utilisation selon la revendication 10, **caractérisée par le fait qu'**elle est présente en une quantité allant de 0,1 à 15% en poids en poids du poids total de la composition.

12. Utilisation selon la revendication 11, **caractérisée par le fait qu'**elle est présente en une quantité allant de 0,5 à 10 % en poids du poids total de la composition.

13. Utilisation selon l'une quelconque des revendications précédentes, **caractérisée par le fait que** la composition de pré-traitement se présente sous forme de lotions, gels, crèmes, shampooings, sticks, mousses, sprays .

14. Utilisation selon l'une quelconque des revendications précédentes, **caractérisée par le fait que** la composition de pré-traitement est conditionnée en flacon pompe ou dans un récipient aérosol.

15. Utilisation selon la revendication 14, **caractérisée par le fait que** la composition de pré-traitement est associée à au moins un agent propulseur choisi parmi les alcanes, le diméthyl éther, l'azote, le protoxyde d'azote, le gaz carbonique ou les halogénoalcanes.

16. Utilisation selon l'une quelconque des revendications précédentes, **caractérisée par le fait que** la composition de pré-traitement comprend au moins un agent tensioactif de nature non-ionique, cationique, anionique ou amphotère.

17. Utilisation selon la revendication 16, **caractérisée par le fait que** la composition de pré-traitement comprend un mélange d'agents tensioactifs comprenant au moins un agent tensioactif anionique, le ou les autres agents tensioactifs étant non ioniques ou amphotères.

18. Utilisation selon l'une quelconque des revendications précédentes, **caractérisée par le fait que** la composition de pré-traitement comprend au moins un polymère additionnel autre que la silicone aminoéthyliminoalkyl(C₄-C₈).

19. Utilisation selon la revendication 18, **caractérisée par le fait que** le polymère est de nature non-ionique, cationique, anionique ou amphotère.

20. Utilisation selon la revendication 19, **caractérisée par le fait que** le polymère est une silicone aminée différente de la silicone aminoéthyliminoalkyl(C₄-C₈).

21. Utilisation selon l'une quelconque des revendications précédentes, **caractérisée par le fait que** la composition de pré-traitement présente un pH allant de 2 à 11 et de préférence de 4 à 9.

22. Utilisation selon la revendication 1 pour améliorer la montée de la coloration.

23. Utilisation selon la revendication 22 pour améliorer la montée de la coloration sur des cheveux sensibilisés.

24. Utilisation selon la revendication 23 pour améliorer l'état des fibres, et en particulier après une coloration comprenant un agent oxydant.

25. Utilisation selon la revendication 24 pour diminuer la porosité des fibres.

26. Utilisation selon la revendication 24 pour diminuer la solubilité alcaline des fibres.

27. Utilisation selon la revendication 1 pour améliorer la ténacité de la coloration aux shampooings.

28. Procédé de coloration des fibres kératiniques humaines et plus particulièrement des cheveux, **caractérisé par le fait qu'**il consiste à appliquer sur les fibres, dans une première étape, une composition de pré-traitement comprenant au moins une silicone aminoéthyliminoalkyl(C₄-C₈) et telle que décrite à l'une quelconque des revendications 1 à 9, puis, après avoir rincé ou non rincé les fibres, appliquer dans une seconde étape, une composition colorante directe, à la laisser agir pendant un temps suffisant pour développer la couleur, puis rincer ou non rincer les fibres et les sécher.

29. Procédé de coloration des fibres kératiniques humaines et plus particulièrement des cheveux, **caractérisé par le fait qu'**il consiste à appliquer sur les fibres, dans une première étape, une composition de pré-traitement comprenant au moins une silicone aminoéthyliminoalkyl(C₄-C₈) et telle que décrite à l'une quelconque des revendications 1 à 9, puis, après avoir rincé ou non rincé les fibres, appliquer dans une seconde étape, une composition colorante d'oxydation, à la laisser agir pendant un temps suffisant pour développer la couleur, puis rincer ou non rincer les fibres et les sécher.

30. Procédé selon l'une quelconque des revendications 28 ou 29, **caractérisé par le fait que** la composition de pré-traitement est posée pendant un temps allant de quelques secondes à 60 minutes et de préférence de 30 secondes à 15 minutes.
